# EUROPÄISCHE PATENTSCHRIFT

(11) **EP 3 355 268 B1**
(45) Veröffentlichungstag und Bekanntmachung des Hinweises auf die Patenterteilung: **26.07.2023**
(21) Anmeldenummer: 18153407.4
(22) Anmeldetag: 25.01.2018
(51) Int. Cl.: G06T 5/00, G06T 7/73

(54) **VERFAHREN, RECHENEINRICHTUNG UND SYSTEM ZUR VERMESSUNG EINER RÖNTGENAUFNAHME EINES MEDIZINISCHEN UNTERSUCHUNGSBEREICHS**
METHOD, COMPUTING APPARATUS AND SYSTEM FOR MEASURING AN X-RAY RECORDING OF A MEDICAL EXAMINATION AREA
PROCÉDÉ, DISPOSITIF DE CALCUL ET SYSTÈME DE MESURE D'UNE RADIOGRAPHIE D'UNE ZONE D'EXAMEN MÉDICALE

(30) Priorität: 25.01.2017 DE 102017201164
(43) Veröffentlichungstag der Anmeldung: 01.08.2018
(73) Patentinhaber: Raylytic GmbH, 04109 Leipzig (DE)
(72) Erfinder: Trautwein, Frank Thilo, 70794 Filderstadt (DE); Dreischarf, Marcel, 04157 Leipzig (DE)
(74) Vertreter: Glawe, Delfs, Moll

(56) Entgegenhaltungen:
- ZHENG G ET AL: "HipMatch: An object-oriented cross-platform program for accurate determination of cup orientation using 2D-3D registration of single standard X-ray radiograph and a CT volume", COMPUTER METHODS AND PROGRAMS IN BIOMEDICINE, ELSEVIER, AMSTERDAM, NL, Bd. 95, Nr. 3, 1. September 2009 (2009-09-01), Seiten 236-248, XP026250765, ISSN: 0169-2607, DOI: 10.1016/J.CMPB.2009.02.009 [gefunden am 2009-03-27]
- Lorenz Fieten ET AL: "Surface-based determination of the pelvic coordinate system", Proceedings of SPIE, vol. 7261, 26 February 2009 (2009-02-26), page 726138, XP055744089, US ISSN: 0277-786X, DOI: 10.1117/12.812321
- Syoji Kobashi ET AL: "Robust Pelvic Coordinate System Determination for Pose Changes in Multidetector-row Computed Tomography Images ?? (Authors) Robust Pelvic Coordinate System Determination for Pose Changes in Multidetector-row Computed Tomography Images", INTERNATIONAL JOURNAL of FUZZY LOGIC and INTELLIGENT SYSTEMS, 1 January 2010 (2010-01-01), pages 65-72, XP055744086, Retrieved from the Internet: URL:http://www.dbpia.co.kr/journal/article Detail?nodeId=NODE01394284
- FOROUGHI PEZHMAN ET AL: "Localization of Pelvic Anatomical Coordinate System Using US/Atlas Registration for Total Hip Replacement", 6 September 2008 (2008-09-06), BIG DATA ANALYTICS IN THE SOCIAL AND UBIQUITOUS CONTEXT : 5TH INTERNATIONAL WORKSHOP ON MODELING SOCIAL MEDIA, MSM 2014, 5TH INTERNATIONAL WORKSHOP ON MINING UBIQUITOUS AND SOCIAL ENVIRONMENTS, MUSE 2014 AND FIRST INTERNATIONAL WORKSHOP ON MACHINE LE, XP047462446, ISBN: 978-3-642-17318-9

## Beschreibung

Die Erfindung betrifft ein Verfahren sowie eine Recheneinrichtung und ein System zur Vermessung einer Röntgenaufnahme eines medizinischen Untersuchungsbereichs.

Medizinische Röntgenaufnahmen werden häufig zur Diagnose, OP-Planung, zur Herstellung patientenspezifischer Implantate und zur quantitativen Beurteilung des Krankheits- oder Behandlungsverlaufs vermessen.

Zur Vermessung von Röntgenaufnahmen stehen heutzutage hauptsächlich computergestützte Verfahren zur Verfügung. Diese beschränken sich jedoch in der Praxis auf die Vermessung von Abständen, Winkeln oder Flächeninhalten in einer Röntgenaufnahme mit Hilfe von "digitalen Linealen" am Computerbildschirm. Hierfür werden üblicherweise zwei Strukturen durch entsprechende Punkte oder Landmarken am Bildschirm identifiziert, mit einem Eingabegerät, z.B. einer Maus mit der Röntgenaufnahme registriert, und bei bestimmten Messungen eine Richtung vorgegeben in die die Abstandsmessung hinsichtlich ihrer x- und y-Komponente zerlegt wird.

An 3D-Modellen sind Messungen prinzipiell auf die gleiche Weise möglich, erfordern jedoch hier die Auswahl der passenden Schnittebene. Sollte die Messebene nicht in einer vom bildgebenden Gerät oder von der anschließenden Rekonstruktion vorgegebenen Schnittebene liegen, muss der Anwender jeweils eine davon abweichende Ebene durch eine "multiplanare Reformation" (MPR) definieren. Aufgrund der hohen Strahlenbelastung durch eine CT-Aufnahme, die schlechte Bildgebung knöcherner Strukturen bei MRT-Aufnahmen, und die praktisch nicht gegebene Möglichkeit zur Darstellung von sogenannten Funktionsaufnahmen (Röntgenaufnahmen, die in einer oder mehreren vorgegebenen Körperhaltungen aufgenommen werden) in den 3D-Modalitäten, sind Röntgenaufnahmen bei vielen Fragestellungen nach wie vor die erste Wahl als bildgebendes Diagnoseinstrument. Diese Funktionsaufnahmen werden häufig zur Diagnose und Beurteilung des Behandlungsverlaufs angefertigt und dienen der Angabe der Bewegung eines zweiten Objekts (z.B. eines Wirbelkörpers) relativ zu einem Ersten. Dazu muss sich der Patient beispielsweise einmal nach vorne beugen (Flexion), und für eine zweite Aufnahme nach hinten.

Aufgrund geringer Abweichungen der Ausrichtung des Patienten relativ zum Röntgengerät und innerer Unsymmetrien der interessierenden Objekte (z.B. hervorgerufen durch eine Skoliose bei der Wirbelsäule) sind diese Funktionsaufnahmen in vielen Fällen nicht exakt senkrecht zu den Wirbelkörpern aufgenommen, so dass die resultierenden Bilder jeweils eine unterschiedliche Abbildung der selben Wirbel zur Folge haben. Aufgrund der Variabilität der Röntgenaufnahme infolge von Verkippungen der Aufnahmeebene ("Out-of-Plane") gegenüber der Objektebene, die durch das oder die zu analysierenden Objekte definiert wird, ist die korrekte Auswahl der Messpunkte bei vergleichenden Funktionsaufnahmen schwierig und häufig fehlerbehaftet.

Aber auch bei statischen Einzelaufnahmen können Röntgenaufnahmen durch Fehler bei der Positionierung des Patienten oder durch krankhafte Deformitäten (z.B. Skoliose) häufig nicht genau senkrecht und reproduzierbar zu den zu vermessenden Objekten (Knochen, Wirbel, Organe, Gefäße, Tumore) aufgenommen werden.

Dadurch entzieht sich bis dato die Analyse klinischer Bilddaten weitgehend einer automatisierten Auswertung und Weiterverarbeitung. Die Verkippungen führen außerdem zu einer Verzerrung von Winkeln und einer Verkürzung der gemessenen Abstände gegenüber den tatsächlichen Abständen, so dass eine große Anzahl der Messungen an radiologischen Bildern nur von eingeschränktem Wert für diagnostische und statistische Zwecke oder zur Anfertigung patientenspezifischer Implantate sind. Die Größenbestimmung oder Anpassung von Implantaten an die morphologischen Verhältnisse ist anhand von 2D-Abbildungen häufig nicht möglich und erfordert eine 3D-Bildgebung mit aufwendigerer Rekonstruktion und Planung durch den Anwender.

Demgegenüber werden erfindungsgemäß ein Verfahren mit den Merkmalen des Anspruchs 1, eine Recheneinrichtung mit den Merkmalen des Anspruchs 12, eine Vorrichtung mit den Merkmalen des Anspruchs 13, ein Computerprogramm mit den Merkmalen des Anspruchs 14 sowie ein System mit den Merkmalen des Anspruchs 15 vorgeschlagen. Vorteilhafte Ausführungsformen sind in den Unteransprüchen angegeben.

Mit Hilfe der Erfindung können zweidimensionale medizinische Abbildungen menschlicher Organe, Gefäße, Tumore oder Knochen, die nicht lotrecht, also nicht exakt entlang oder senkrecht zu einer Achse oder Symmetrieebene des interessierenden Objekts oder der unmittelbaren Umgebung aufgenommen wurden, exakt vermessen werden.

Es werden zunächst einige im Rahmen der Erfindung verwendete Begriffe erläutert.

Ein medizinischer Untersuchungsbereich kann ein beliebiger Bereich des menschlichen (oder tierischen) Körpers sein. Ein vom Untersuchungsbereich umfasstes Objekt kann grundsätzlich ein beliebiger Teilbereich des Untersuchungsbereiches sein. Beispielsweise kann ein Objekt ein Knochen, Wirbel, Organ, Gefäß, oder Tumor sein oder jeweils durch einen Teilabschnitt der vorgenannten Elemente gebildet sein.

Eine Röntgenaufnahme des medizinischen Untersuchungsbereiches ist eine mit Hilfe einer Röntgenvorrichtung gewonnene Abbildung des Untersuchungsbereiches, wobei die Abbildung eine (reale) Projektionsrichtung sowie eine (reale) Aufnahmeebene aufweist, welche üblicherweise orthogonal zur Projektionsrichtung ausgerichtet ist. Die Lage der Aufnahmeebene ist beispielsweise definiert durch die Lage des Röntgendetektors bzw. der Filmkassette der Röntgenvorrichtung. Die Röntgenaufnahme kann in digitaler Form vorliegen oder in eine digitale Form umgewandelt werden.

Ein digitales rekonstruiertes Röntgenbild kann durch eine Simulation gewonnen werden, bei der eine virtuelle Projektionsrichtung sowie eine virtuelle Aufnahmeebene angenommen werden, wobei die virtuelle Aufnahmeebene bevorzugt orthogonal zur virtuellen Projektionsrichtung ausgerichtet ist.

Ein 3D-Modell des Untersuchungsbereiches stellt eine virtuelle dreidimensionale Repräsentation des Untersuchungsbereiches dar. Das 3D-Modell umfasst die 3D-Objekte, wobei die 3D-Objekte jeweils eine virtuelle dreidimensionale Repräsentation des jeweiligen Objektes darstellt.

Erfindungsgemäß wird eine Objektebene ermittelt, welche einem der 3D-Objekte zuzuordnen ist. Auch das reale Objekt weist eine entsprechende Objektebene auf. Die Objektebene ist bevorzugt so ausgerichtet, dass eine senkrecht zur Objektebene aufgenommene Röntgenaufnahme Messergebnisse mit großer Genauigkeit liefert. Die genaue Orientierung der Objektebene hängt regelmäßig ab von der Art des untersuchten Objektes sowie von der Art der gesuchten Messergebnisse. Üblicherweise ist die Objektebene symmetrisch innerhalb des Objektes angeordnet. Die Ausrichtung der Objektebene kann durch morphologische Merkmale (Kanten, Flächen, Symmetrieebenen) des Objekts bestimmt werden. Soll beispielsweise die Länge eines Wirbelkörpers in anterior-posteriorer Richtung gemessen werden, so kann die Objektebene als Symmetrieebene mittig durch den Wirbelkörper verlaufen (idealisiert entsprechend der Sagittalebene des menschlichen Körpers).

Die vorliegende Erfindung macht sich zunutze, dass im Laufe der Diagnose oder Behandlung per Computertomographie (CT) oder Magnetresonanztomographie (MRT) häufig 3D-Abbildungen eines Untersuchungsbereiches sowie der interessierenden Objekte erstellt werden. Aus diesen 3D-Abbildungen kann das 3D-Modell sowie das darin enthaltene 3D-Objekt erstellt werden. Alternativ kann das 3D-Modell und/oder das 3D-Objekt durch eine mathematische, dreidimensionale, diskrete Beschreibung eines oder mehrerer der interessierenden Objekte generiert werden, wobei die 3D-Objekte in dem (gemeinsamen) 3D-Modell positioniert sind. Bevorzugt werden das 3D-Modell und/oder die 3D-Objekte mit physikalischen Eigenschaften zur Berechnung der Abschwächung eines das 3D-Modell bzw. die 3D-Objekte durchdringenden (virtuellen) Röntgenstrahls versehen.

Das bereitgestellte 3D-Modell wird anschließend zur Berechnung des digital rekonstruierten Röntgenbildes verwendet, wobei die virtuelle Projektionsrichtung verändert wird, bis eine maximale Übereinstimmung zwischen der Röntgenaufnahme und dem digital rekonstruierten Röntgenbild besteht. Der Begriff "maximale Übereinstimmung" bedeutet nicht, dass eine absolute oder vollständige Übereinstimmung notwendig ist. Es kann ausreichen, wenn Übereinstimmung bis zu einem gewünschten Grad besteht. Die Ermittlung der maximalen Übereinstimmung wird nachfolgend auch 2D-3D-Registrierung genannt.

Die Ermittlung der maximalen Übereinstimmung stellt eine Lösung eines mathematischen Optimierungsproblems dar. Der Grad der Übereinstimmung zwischen den Bildern wird berechnet, wobei ein Ziel darin besteht, die Richtung der höchsten Übereinstimmung des projizierten Bildes mit dem Referenzbild zu finden. Derartige Verfahren zur multi-variablen Optimierung sind dem Fachmann wohlbekannt und bspw. beschrieben in "Medical image registration: a review" von Francisco P.M. Oliveira und Joa~o Manuel R.S. Tavares, Institute de Engenharia Meca^nica e Gesta~o Industrial, Faculdade de Engenharia, Universidade do Porto, Rua Dr. Roberto Frias, 4200-465 Porto, Portugal, oder in "Evaluation of optimization methods for intensity-based 2D-3D registration in x-ray guided interventions," von I.M.J. van der Boma, S. Kleinb, M. Staringc, R. Homand, L.W. Bartelse, und J.P.W. Pluime.

Indem durch das erfindungsgemäße Verfahren sowohl eine 2D-3D-Registrierung vorgenommen wird, als auch eine Objektebene des interessierenden 3D-Objektes ermittelt wird, kann die Röntgenaufnahme durch das erfindungsgemäße Verfahren auf besonders einfache und exakte Weise vermessen werden.

Dies wird durch die Definition der korrigierten Projektionsrichtung und der zugehörigen virtuellen Ausgabeebene erreicht, welche in einer bekannten Ausrichtung zur ermittelten Objektebene steht. Insbesondere dann, wenn die reale Röntgenaufnahme in einem nicht exakt orthogonalen Winkel zur Objektebene aufgenommen wurde, liefert das Verfahren eine an der Objektebene orientierte korrigierte Projektionsrichtung sowie eine zugehörige Ausgabeebene, welche eine korrigierte Vermessung des Röntgenbildes erlaubt.

Aus "Zheng, G., Zhang, X., Steppacher, S.D., Murphy, S.B., Siebenrock, K.A., Tannast, M., 2009. HipMatch: An object-oriented cross-platform program for accurate determination of cup orientation using 2D-3D registration of single standard X-ray radiograph and a CT volume. Computer Methods and Programs in Biomedicine 95, 236-248." ist ein Verfahren bekannt, das die Objektebene durch die manuelle Platzierung von Landmarken im 3D-Objekt festlegt.

Kern der Erfindung ist somit die Korrektur zweidimensionaler medizinischer Röntgenaufnahmen (beispielsweise von Gefäßen, Tumoren oder Knochen), die nicht lotrecht, also nicht exakt entlang oder senkrecht zu einer Achse oder Symmetrieebene der interessierenden Objekte oder der unmittelbaren Umgebung aufgenommen wurden und dadurch eine genaue Vermessung der Objekte erschweren. Zur Korrektur der zweidimensionalen Abbildung wird ein 3D-Modell verwendet, das zumindest zwei der interessierenden Objekte beinhaltet. Bevorzugt wird anhand des 3D-Modells eine Objektebene definiert und die Verkippung der Aufnahmeebene (bzw. der unkorrigierten Projektionsrichtung) relativ zur Objektebene ermittelt. Mit dieser Information kann die Messung beliebiger Abstände, Winkel oder Flächen in der ursprünglichen Abbildung korrigiert werden, alternativ eine auf dem geometrisch ggf. modifizierten 3D-Modell basierte, digital rekonstruierte Abbildung in der gewünschten Objektebene erzeugt werden.

Das erfindungsgemäße Verfahren erlaubt es somit, Messungen von Winkeln, Abstand und Flächeninhalt an potentiell "verkippten" Röntgenaufnahmen mit hoher Präzision durchzuführen, wobei der Aufwand für manuelle Benutzerinteraktion gering ist. An einzelnen Röntgenaufnahmen sind Absolutmessungen von Abstandsmaßen mit hoher Genauigkeit möglich, indem der bekannte Absolutmaßstab des 3D-Modells (Voxel/mm) auf das rekonstruierte Röntgenbild und/oder die ursprüngliche Röntgenaufnahme (100) projiziert werden kann (Pixel/mm).

Das Verfahren kann zudem mit einer Mehrzahl von Röntgenaufnahmen durchgeführt werden, so dass an mehreren Röntgenaufnahmen vergleichende Relativmessungen durchgeführt werden können. Ein Abstandsmaß wird in diesem Fall vorzugsweise auf den Abstand in einem anderen Objekt einer anderen Röntgenaufnahme bezogen und das Verhältnis angegeben.

Bevorzugt ist die Ausgabeebene parallel zu der ermittelten Objektebene ausgerichtet. Dies ist gleichbedeutend damit, dass die korrigierte Projektionsrichtung senkrecht bzw. im wesentlichen senkrecht zur Objektebene ausgerichtet ist.

Alternativ kann die Ausgabeebene auch parallel zu einer Hauptebene eines durch das 3D-Modell oder durch das 3D-Objekt vorgegebenen Koordinatensystems ausgerichtet sein. Dies ist besonders dann vorteilhaft, wenn das 3D-Modell an einem liegenden Patienten erzeugt wurde, da der Patient in diesem Fall bei der Erstellung des 3D-Modells bzw. der 3D-Objekte für viele Messaufgaben bereits nahezu optimal und reproduzierbar ausgerichtet ist, so dass sich die Objektebenen der 3D-Objekte bereits in einer optimalen Ausrichtung relativ zum 3D-Modell befinden.

In einer vorteilhaften Ausführungsform umfasst der Schritt des Vermessens der Röntgenaufnahme das Ermitteln einer Korrekturfunktion, welche sich aus der Abweichung zwischen korrigierter virtueller Projektionsrichtung und der ermittelten Projektionsrichtung ergibt, und das Anwenden der Korrekturfunktion auf die gewonnenen Messergebnisse.

In diesem Fall kann daher eine Messung unmittelbar an der (realen) Röntgenaufnahme vorgenommen werden, wobei etwaige Messungenauigkeiten, welche aus einer Verkippung der Objektebene relativ zur realen Projektionsrichtung bei der Erstellung der Röntgenaufnahme entstanden sind, durch die erfindungsgemäße Korrekturfunktion korrigiert werden.

Alternativ kann vorgesehen sein, dass in dem Schritt des Vermessens anhand der korrigierten virtuellen Projektionsrichtung ein korrigiertes digital rekonstruiertes Röntgenbild in der Ausgabeebene erzeugt wird, wobei die Röntgenaufnahme unter Verwendung des korrigierten digital rekonstruierten Röntgenbildes in der Ausgabeebene vermessen wird. Die Messung kann in diesem Fall unmittelbar am digital rekonstruierten Röntgenbild vorgenommen werden, welches unter Verwendung einer korrigierten Projektionsrichtung in die Ausgabeebene projiziert wird.

Bevorzugt umfasst der Schritt des Vermessens die Messung eines Abstandes zwischen Messpunkten, die Messung eines Winkels zwischen Messgeraden und/oder die Messung eines Flächeninhalts von Messflächen.

Es kann vorgesehen sein, dass das 3D-Objekt durch ein Verfahren zur dreidimensionalen Bildgebung, insbesondere durch Computertomographie oder Magnetresonanztomographie, gewonnen ist. In diesem Fall kann die Objektebene ermittelt werden auf Basis des 3D-Objekts unter Verwendung von dem 3D-Objekt zugeordneten Grauwertintensitäten. Bevorzugt wird die virtuelle Objektebene auf Basis einer Optimierung, insbesondere einer Least-Square-Optimierung, des Abstandes zwischen Punkten, Geraden, Kurven oder Ebenen und einer Auswahl von Voxeln des 3D-Objekts ermittelt und/oder auf Basis von Kanten oder Flächen des 3D-Objekts und/oder mit Methoden des maschinellen Lernens, insbesondere mit trainierten neuronalen Netzwerken.

Alternativ kann das 3D-Objekt auf einer computergenerierten Geometriebeschreibung basieren, beispielsweise auf einem "Statistical Shape Modell", einem "Active Appearance Modell" oder einem "Active Shape Modell". In diesem Fall kann das erfindungsgemäße Verfahren ausgeführt werden, auch wenn keine dreidimensionalen Aufnahmen von dem Untersuchungsbereich vorliegen.

Es kann vorgesehen sein, dass die virtuelle Objektebene ermittelt wird auf Basis von vorab mit dem 3D-Objekt assoziierten Landmarken.

In diesem Fall kann vorgesehen sein, dass zusätzlich das 3D-Objekt durch lokale und/oder elastische Deformation verändert wird. Der Begriff lokale Deformation bezeichnet hier eine Veränderung eines Teilbereichs des 3D-Objektes. Eine elastische Deformation bezeichnet die Veränderung der Gestalt des 3D-Objektes mit Hilfe elastischer Modelle, bei denen das 3D-Objekt beispielsweise als elastische oder zähe Flüssigkeit modelliert wird. Es kann auf diese Weise auch bei komplexen (lokalen) Verzerrungen eine Registrierung zwischen 3D-Objekt und Röntgenaufnahme stattfinden. Eine lokale und/oder elastische Deformation unterscheidet sich insofern von einer affinen Transformation, bei der ein starres 3D-Objekt lediglich verschoben und ausgerichtet wird.

Das erfindungsgemäße Verfahren wird bei einem Untersuchungsbereich verwendet, welcher zumindest zwei Objekte umfasst. Dabei ist vorgesehen, dass innerhalb des 3D-Modells die Positionen und/oder Ausrichtungen der 3D-Objekte (300') relativ zueinander verändert werden,
- die Schritte des Vergleichens und des Veränderns wiederholt werden, bis für jedes 3D-Objekt die Position und/oder die relative Ausrichtung gefunden ist, bei der eine maximale Übereinstimmung zwischen der Röntgenaufnahme und dem digital rekonstruierten Röntgenbild besteht.

Erfindungsgemäß wird eine einem der 3D-Objekte zuzuordnende virtuelle Objektebene ermittelt. Des weiteren kann eine der Mehrzahl von 3D Objekten zuzuordnende virtuelle Objektebene ermittelt werden, wobei die ermittelten Positionen der 3D-Objekte innerhalb des 3D-Modells und/oder die Ausrichtungen der 3D-Objekte relativ zur korrigierten Projektionsrichtung berücksichtigt werden.

Der Schritt des Vermessens kann die Messung eines Abstandes zwischen zwei Messpunkten und/oder die Messung eines Winkels zwischen zwei Messgeraden umfassen, wobei die beiden Messpunkte und/oder Messgeraden bevorzugt verschiedenen 3D-Objekten zuzuordnen sind.

In einer vorteilhaften Ausführungsform wird ein Längenmaßstab des 3D-Modells auf die Röntgenaufnahme übertragen. Dies ist besonders vorteilhaft, da Röntgenaufnahmen häufig unkalibriert bezüglich eines Längenmaßstabs sind, das 3D-Modell jedoch einen kalibrierten Maßstab besitzt. Es wird somit auf einfache Weise eine kalibrierte Messung unmittelbar an der Röntgenaufnahme ermöglicht.

Gegenstand der Erfindung ist zudem eine Vorrichtung zur Durchführung des erfindungsgemäßen Verfahrens mit den Merkmalen des Anspruchs 15. Die Vorrichtung kann durch weitere im Zusammenhang mit dem erfindungsgemäßen Verfahren beschriebene Merkmale fortgebildet werden.

Gegenstand der Erfindung ist auch ein Computerprogramm zur Vermessung einer Röntgenaufnahme eines medizinischen Untersuchungsbereiches, das einen Computerprogrammcode für die Durchführung des erfindungsgemäßen Verfahrens enthält. Das Computerprogramm kann auf einem maschinenlesbaren Medium gespeichert sein (Computerprogrammprodukt).

Nachfolgend werden vorteilhafte Ausführungsformen der Erfindung unter Bezugnahme auf die beigefügten Zeichnungen beispielhaft erläutert.
Figur 1A: zeigt eine Aufsicht auf einen medizinischen Untersuchungsbereich mit leicht verkippter Ausrichtung gegenüber einer Projektionsrichtung während eines aus dem Stand der Technik bekannten Vorgangs zur Erstellung einer Röntgenaufnahme.
Figur 1B: zeigt eine Perspektivische Ansicht von Figur 1A.
Figur 1C: zeigt eine Illustration einer durch die Anordnung der Figuren 1A und 1B gewonnenen Röntgenaufnahme.
Figur 2: zeigt eine Illustration des Verfahrens der 2D-3D-Registrierung aus verschiedenen Projektionsrichtungen.
Figur 3: zeigt eine Illustration einer resultierenden, digital rekonstruierten Röntgenabbildung, deren Projektionsrichtung der Projektionsrichtung der unkorrigierten Röntgenaufnahme entspricht.
Figur 4: zeigt eine Darstellung der 3D-Objekte und der Objektebenen innerhalb des 3D-Modells.
Figur 5: zeigt eine Illustration der korrigierten digitalen Rekonstruktion des Röntgenbildes in der Ausgabeebene.
Figur 6: zeigt eine Illustration eines korrigierten digital rekonstruierten Röntgenbildes in der Ausgabeebene.
Figur 7: zeigt eine Illustration der Ermittlung des Verkippungswinkels bzw. des Differenzwinkels zwischen unkorrigierter und korrigierter Projektionsrichtung.

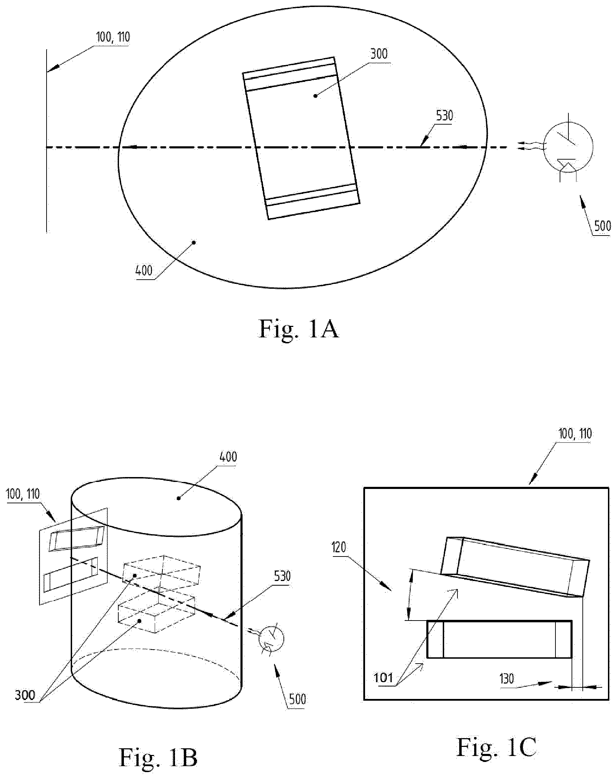

Die Figuren 1A bis 1C illustrieren ein aus dem Stand der Technik bekanntes Verfahren zum Erstellen einer Röntgenaufnahme. Figur 1A zeigt eine Röntgenquelle 500, welche Röntgenstrahlung entlang einer Projektionsrichtung 530 auf einen medizinischen Untersuchungsbereich 400 ausstrahlt, wobei eine Röntgenaufnahme 100 in einer Aufnahmeebene 110 entsteht. Der Untersuchungsbereich 400 weist ein Objekt 300 auf, welches beispielsweise als Wirbelkörper ausgebildet sein kann, wobei eine Symmetrieebene des Objekts 300 relativ zur Projektionsrichtung 530 verkippt ist.

Figur 1B zeigt eine perspektivische Ansicht der Figur 1A, wobei ersichtlich ist, dass der Untersuchungsbereich 400 zwei Objekte 300 aufweist.

Figur 1C zeigt eine vergrößerte Ansicht der Röntgenaufnahme 100 in der Aufnahmeebene 110. Es ist zudem eine Winkelmessung 120 zwischen den beiden Objekten sowie eine Abstandsmessung 130 illustriert. Aufgrund der Verkippung der Objekte 300 relativ zur Projektionsrichtung 530 weist das in die Aufnahmeebene 110 projizierte Bild 101 der Objekte 300 in der Röntgenaufnahme 100 sogenannte "Doppelkanten" auf. In der Praxis führt dies zu einer unscharfen Darstellung der Objektumrisse auf der Röntgenaufnahme 100. Die Winkel- und Abstandsmessungen sind daher mit einem Messfehler behaftet.

Mit Hilfe einer erfindungsgemäßen Vorrichtung sowie unter Anwendung des erfindungsgemäßen Verfahrens kann die Röntgenaufnahme 100 wie nachfolgend beschrieben vermessen werden.

Eine nicht gezeigte beispielhafte Vorrichtung zur Durchführung des erfindungsgemäßen Verfahrens kann dazu einen Speicher, ein Rechenmodul, ein Vergleichsmodul, ein Veränderungsmodul, ein Geometriemodul und ein Vermessungsmodul aufweisen. Die Elemente der erfindungsgemäßen Vorrichtung können durch einen Computer gebildet werden, auf dem die erfindungsgemäße Software ausgeführt wird. Der Computer kann einen Mikroprozessor, einen Arbeitsspeicher und einen nichtvolatilen Speicher oder einer I/O Schnittstelle zu einem externen, nichtvolatilen Speicher umfassen.

In einer besonders vorteilhaften Ausführung ist der Mikroprozessor zur stark parallelisierten Codeausführung geeignet (z.B. ein Grafikprozessor) und der Arbeitsspeicher möglichst direkt und mit möglichst hoher Bandbreite an den Mikroprozessor angebunden (z.B. der RAM einer Grafikkarte).

In einer beispielhaften Ausführungsform des erfindungsgemäßen Verfahrens wird zunächst ein 3D-Modell 400` des Untersuchungsbereiches im Speicher bereitgestellt, welches wenigstens teilweise mit dem medizinischen Untersuchungsbereich 400 korrespondiert. Nachfolgend wird zur Vereinfachung die Anwendung auf lediglich eine Röntgenaufnahme 100 beschrieben, das Verfahren ist jedoch genauso auf mehrere Röntgenaufnahmen 100 anwendbar, wie sie z.B. bei stereoskopischen oder biplanaren Aufnahmen entstehen.

Anschließend können, sofern nicht die kompletten Bildinhalte verglichen werden, die relevanten Bereiche in der Röntgenaufnahme 100 und/oder des 3D-Modells 400'definiert werden. Es kann dazu ein optionales Auswahlmodul vorhanden sein. Sollen mehrere, zueinander bewegliche Objekte 300 vermessen werden, kann es durch unterschiedliche Orientierung des Patienten (z.B. stehen vs. liegen) oder eines zeitlichen Abstandes zu einer Lageabweichung der 3D-Objekte zueinander zwischen der Röntgenaufnahme 100 und dem 3D-Modell 400` gekommen sein. In diesem Fall kann mit Hilfe des Auswahlmoduls eine Zuordnung zwischen den auf der Röntgenaufnahme 100 abgebildeten Objekten 300 und den vom3D-Modell 400` umfassten 3D-Objekten 300` vorgenommen werden.

Nachfolgend werden mit Hilfe des Rechenmoduls unter Verwendung des 3D-Modells 400` und der ggf. zuvor selektierten Objekte oder Bereiche, digital rekonstruierte Röntgenbilder (DRR) 520 des 3D-Modells 400` bzw. der 3D-Objekte 300` des Patienten aus unterschiedlichen Projektionsrichtungen 540 berechnet. Dies geschieht im Ausführungsbeispiel mit Hilfe einer virtuellen Röntgenquelle 510 und einer physikalisch-mathematischen Beschreibung der Röntgenstrahlabschwächung eines von Röntgenstrahlen durchdrungenen 3D-Modells sowie einer Projektionsebene, auf welcher die nach der Abschwächung durch den Körper verbleibende Strahlung diskretisiert berechnet und in grauwerten dargestellt wird.

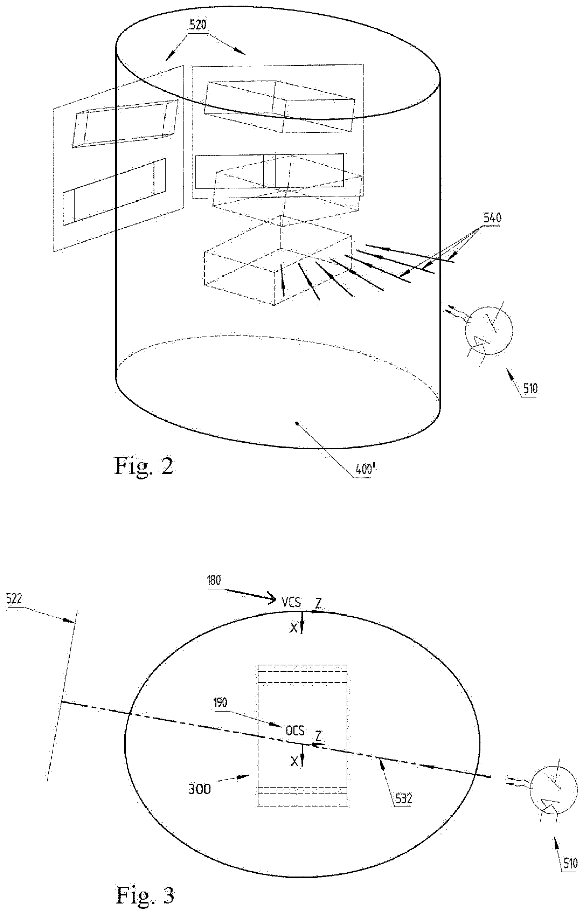

Die Ähnlichkeit der digital rekonstruierten Röntgenbilder (DRR) 520 wird in einem Vergleichsmodul für den selektierten Bildausschnitt der Röntgenaufnahme 100 bewertet. Das Veränderungsmodul kann anschließend beispielsweise über einen Optimierungsalgorithmus die Projektionsrichtung gegenüber dem 3D-Modell 400` und/oder gegenüber den 3D-Objekten 300` variieren, bis eine maximale Übereinstimmung zwischen einem digital rekonstruierten Röntgenbild (DRR) 520 und der Röntgenaufnahme 100 besteht. Diese so gefundene unkorrigierte Projektionsrichtung 532 liefert ein unkorrigiertes digital rekonstruiertes Röntgenbild 522, welches der Röntgenaufnahme 100 in der Aufnahmeebene (110) entspricht. Der oben beschriebene Vorgang ist rein illustrativ in den Figuren 2 und 3 gezeigt. In Figur 2 ist in einer hypothetischen perspektivischen Ansicht eine virtuelle Röntgenquelle 510 dargestellt, welche mit Hilfe von verschiedenen Projektionsrichtungen 540 eine Mehrzahl von DRRs 520 erzeugt. Figur 3 zeigt den Gegenstand der Figur 2 in einer hypothetischen Aufsicht, wobei die ermittelte unkorrigierte Projektionsrichtung 532 gezeigt und das resultierende unkorrigierte DRR 522 angedeutet ist. Es ist zudem zu sehen, dass die unkorrigierte Projektionsrichtung 532 relativ zu einer Symmetrieebene des Objekts 300` sowie relativ zu einem Objektkoordinatensystem 190 des Objekts 300` verkippt ist.

Sind mehrere Bereiche definiert, kann der zuvor beschriebene Schritt für jeden Bereich wiederholt werden, um die unkorrigierte Projektionsrichtung 532 nicht nur auf einen Bereich, sondern über alle Bereiche mitteln zu können. Die so gegenüber dem 3D-Modell 400` bzw. gegenüber den 3D-Objekten 300` ermittelte unkorrigierte Projektionsrichtung 532 entspricht der realen Projektionsrichtung, welche bei der Erstellung der Röntgenaufnahme verwendet wurde, wobei die reale Projektionsrichtung orthogonal zur Aufnahmeebene 110 der Röntgenaufnahme 100 ausgerichtet ist.

Basiert das 3D-Modell 400` auf einer synthetischen Beschreibung der 3D-Objekte 300', z.B. durch ein computergeneriertes "Statistical Shape Model" (SSM), "Active Appearance Model" (AAM) oder ein "Active Shape Model" (ASM), liegt die Geometriebeschreibung in Form von diskreten Flächen- oder Volumenelementen vor. Dieses Modell kann unabhängig von den vorangegangenen Schritten auf die tatsächliche Morphologie des Patienten angepasst werden. Hierzu ist die Röntgenaufnahme 100 für viele Anwendungen bereits ausreichend. Liegen weitere Röntgenaufnahmen 100 aus anderen Projektionsrichtungen vor, können diese zur Anpassung der mathematischen Geometriebeschreibungen und Verbesserung der Abbildungsgenauigkeit der 3D-Objekte 300` ebenfalls verwendet werden. Die Erzeugung eines patientenspezifischen 3D-Modells 400` mit patientenspezifischen 3D-Objekten 300` auf Basis von Statistical Shape Models und Röntgenaufnahmen ist z.B. durch Zheng et. al. in "Scaled, patient-specific 3D vertebral model reconstruction based on 2D lateral fluoroscopy", Int J CARS (2011) 6:351-366 beschrieben und nicht erfindungswesentlich.

Für den Fall, dass die Messaufgabe die Bestimmung von Winkeln oder Abständen zwischen mehreren, zueinander beweglichen Objekten 300 betrifft, z.B. zwischen Wirbeln der Wirbelsäule, ist das erfindungsgemäße Verfahren ebenfalls geeignet. In diesem Fall wird für jedes 3D-Objekt 310 in einem weiteren Schritt das (ggf. segmentierte) 3D-Modell 400` über eine affine Transformation der 3D-Objekte 300` mit der zuvor beschriebenen 2D-3D-Registrierung iterativ angepasst. Allerdings wird nun nicht die Projektionsrichtung variiert, sondern die Position und Ausrichtung der 3D-Objekte 300` werden so lange variiert, bis das nach jeder Veränderung erzeugte DRR mit der Röntgenaufnahme 100 bestmöglich übereinstimmt. Bevorzugt können Zwangsbedingungen über eine Schnittstelle eingegeben werden, z.B. hinsichtlich der maximalen Translation oder Rotation, um den Suchraum signifikant einzuschränken, so dass diese Anpassung in sehr kurzer Rechenzeit erfolgen kann. Nach Abschluss der Anpassung haben die definierten Ausschnitte bzw. die gesamte Röntgenaufnahme 100 der Objekte 300 und das DRR für die noch unkorrigierte Ausrichtung 522 eine größtmögliche Übereinstimmung. Die 3D-Objekte 300` sind damit korrekt innerhalb des 3D-Modells 400' zueinander und zur Aufnahmeebene 110 angeordnet.

Liegen zeitgleich aufgenommene Röntgenaufnahmen aus unterschiedlichen Aufnahmerichtungen vor (stereo- oder biplanare Aufnahmen), eignen sich diese Röntgenaufnahmen 100 zur besonders genauen Transformation und Anordnung der 3D-Objekte 300` im 3D-Modell 400` nach der zuvor beschriebenen Methode.

In einem weiteren Schritt wird dann mit Hilfe des Geometriemoduls die Ausgabeebene 600 bestimmt, welche der eigentlich gewünschten Messebene entspricht. Diese Ebene kann in einer ersten Variante eine Ebene des Volumenkoordinatensystems (180) (VCS) des 3D-Modells 400' sein (vgl. Figur 3). Dies ist besonders dann vorteilhaft, wenn 3D-Modell 400` bzw. 3D-Objekt 300` bereits eine optimale und reproduzierbare Ausrichtung aufweisen, beispielsweise wenn das 3D-Modell 400` aus einer MRT- oder CRT-Aufnahme an einem liegenden Patienten erzeugt wurde, welcher in diesem Fall für viele Messaufgaben bereits nahezu optimal und reproduzierbar ausgerichtet ist.

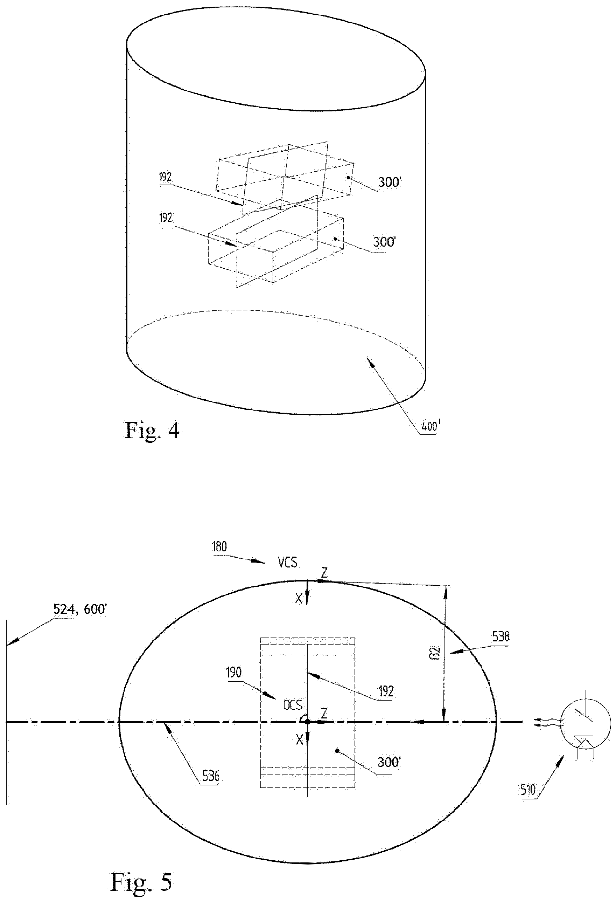

Eine vorteilhafte Ausführung ist die automatische Bestimmung der Ausgabeebene 600 bzw. eines zur Ausgabeebene korrespondierenden Messkoordinatensystems 610 basierend auf der Definition des Objektkoordinatensystems 190 (OCS) oder der Objektebene 192 (vgl. Figuren 3 und 4). Das Objektkoordinatensystem 190 oder die Objektebene 192 wird hierzu zunächst mit Hilfe des erfindungsgemäßen Verfahrens ermittelt. Beispielhafte Objektebenen 192 der 3D-Objekte 300` innerhalb des 3D-Modells 400` sind in Figur 4 gezeigt. Die Ermittlung kann vorteilhafterweise durch eine Auswertung der Grauwertinformationen der 3D-Objekte 300` erfolgen. Dadurch ist insbesondere bei einer unsymmetrischen Anordnung innerer Organe oder Knochen eine Definition anhand der tatsächlichen Lage und Position der relevanten 3D-Objekte 300` möglich. Die Auswahl der Voxel die zur Bestimmung der Objektebene 192 bzw. des Objektkoordinatensystems 190 dienen, kann dazu manuell oder programmatisch eingeschränkt werden. Dies kann, je nach zu identifizierender Struktur, über eines oder mehrere der folgenden Verfahren erreicht werden: a) Einschränkung des zulässigen Grauwertebereichs der Voxel; b) Definition einer oder mehrerer Auswahlbereiche über die Röntgenaufnahme 100; c) über Methoden des maschinellen Lernens (Neuronale Netzwerke); d) Definition einer oder mehrerer Schnittebenen über die Röntgenaufnahme 100 oder e) über das 3D-Modell 400'. Soll die Auswahl der Bereiche bzw. Schnittebenen manuell erfolgen, geschieht dies bevorzugt in der noch unkorrigierten Röntgenaufnahme 100, wobei die Selektion über die unkorrigierte Projektionsrichtung 532 in das 3D-Modell 400` projiziert wird und nur noch die Voxel ausgewählt werden, die durch die Schnittebene, Auswahlbereiche, Graustufen und sonstige Einschränkungen übrigbleiben. Diese Auswahl kann manuell durch den Bediener oder programmatisch erfolgen.

Punkte, Geraden, Ellipsen, Ebenen oder andere analytisch beschreibbare Konturen, zusammengefasst Referenzobjekte eignen sich zur Definition der Objektebene 192 bzw. des Objektkoordinatensystems 190. Die Lage und Form der Referenzobjekte innerhalb des 3D-Modells 400` kann insbesondere I) durch Zentrums- oder (Flächen-) Schwerpunktbetrachtungen; II) Regressionsverfahren wie die Minimierung der Abstandsquadrate von Voxeln zu den Referenzobjekten; III) durch Symmetriebetrachtungen oder IV) durch trainierte neuronale Netzwerke in Verbindung mit zwei- oder dreidimensionalen Geometriebeschreibungen (z.B. AAM, ASM, SSM) erfolgen. Die Referenzobjekte können je nach Messaufgabe bzw. Objekt auch zu Schablonen angeordnet sein, die manuell oder automatisch auf die sichtbaren Strukturen gezogen und angepasst werden können.

Wird das Messkoordinatensystem oder die Ausgabeebene 600 auf Basis der 3D-Objekte 300` definiert, kann es je nach Aufgabenstellung vorteilhaft sein, dies auf Basis des Objektkoordinatensystems 190 oder der Objektebene 192 eines einzelnen 3D-Objekts 300 zu tun, oder auf Basis mehrerer 3D-Objekte 300. Werden mehrere 3D-Objekte 300 zur Definition genutzt, wird die Lage der Ausgabeebene 600 oder des Messkoordinatensystems zweckmäßigerweise durch eine Mittelung der einzelnen Objektebenen 192 oder Objektkoordinatensysteme 190 der einzelnen 3D-Objekte 300, z.B. durch die jeweiligen Winkelhalbierenden zwischen den Richtungsvektoren, definiert. In einer anderen bevorzugten Variante wird das Messkoordinatensystem oder die Ausgabeebene 600 auf Basis beider Objekte definiert (z.B. durch die Symmetrieachse eines superioren und den Mittelpunkt eines inferioren Wirbelkörpers).

Eine ebenfalls vorteilhafte Variante zur Definition der Ausgabeebene 600 oder des Messkoordinatensystems besteht in der Nutzung von vordefinierten Knoten eines mathematischen 3D-Modells 300`, z.B. eines ASM, AAM oder SSM. So kann die Ausgabeebene 600 durch drei charakteristische Punkte vollständig beschrieben sein. Definieren die Punkte gleichzeitig Ursprung und zwei Achsen, ist sogar die Definition des Ausgabekoordinatensystems mit nur drei Punkten möglich.

Ist das Messkoordinatensystem oder die Ausgabeebene 600 nach einer der zuvor beschriebenen Methoden definiert, ist damit gleichzeitig die Ausrichtung der korrigierten Projektionsrichtung 536 als Normalvektor der Ausgabeebene 600 bzw. durch Verwendung der Z-Achse des Messkoordinatensystems definiert. Figur 5 illustriert die korrigierte Projektionsrichtung (536), welche orthogonal zur Objektebene 192 des 3D-Objektes 300` ausgerichtet ist und welche ein korrigiertes DRR 524 in der Ausgabeebene 600 liefert.

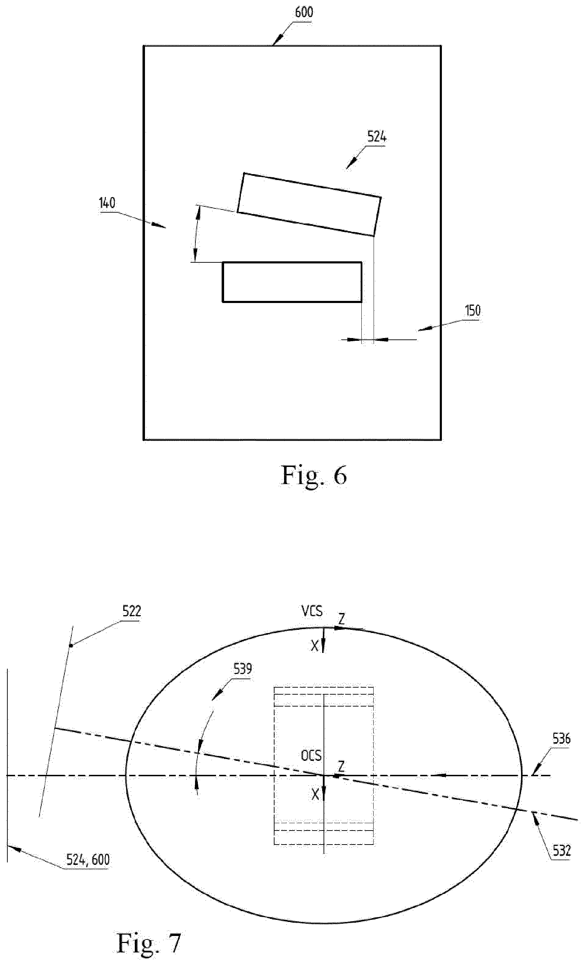

Nachdem nun sowohl die Orientierung der Röntgenaufnahme 100 über die Simulation des DRR 522 der unkorrigierten Ausrichtung 532 , als auch die Ausrichtung der Ausgabeebene 600 bzw. das Messkoordinatensystem bekannt ist, kann in eine weiteren Schritt die Verkippung als Differenz zwischen der Orientierung der Röntgenaufnahme 100 und der korrigierten Ausgabeebene 600 bestimmt werden. Diese Verkippung resultiert aus der Abweichung zwischen der korrigierten Projektionsrichtung 536 und der zuvor ermittelten unkorrigierten Projektionsrichtung 532. Der Vorgang der Ermittlung einer Abweichung zwischen den Projektionsrichtungen 536 und 532 ist in Figur 7 illustriert. Die Abweichung kann in Form eines Differenzwinkels 539 gemessen werden. Das erfindungsgemäße Vermessungsmodul kann dazu ausgestaltet sein, die Abweichung zu ermitteln.

Vor der eigentlichen Vermessung, kann in einem optionalen weiteren Schritt eine Definition der Messpunkte zur Abstandsmessung, der Geraden zur Winkelmessung oder der Flächen zur Flächenmessung erfolgen. Soll die Abstands- oder Winkelmessung auf eine bestimmte Richtung bezogen sein, muss diese Richtung und damit das Messkoordinatensystem ebenfalls definiert werden (falls noch nicht erfolgt). Die Definition kann dabei
- anhand der unkorrigierten Röntgenaufnahme 100 erfolgen;
- in einer vorteilhafteren Ausführung anhand eines korrigierten DRR 524, welches in die Ausgabeebene 600 projiziert wird, oder;
- über vordefinierte Knoten der mathematischen 3D-Objektbeschreibung, z.B. zur Distanzmessung 160 bei ASM, AAM oder SSM.

Vorteil der Variante b) ist die stets senkrechte bzw. gleichmäßige Abbildung der zu untersuchenden Strukturen, was die manuelle oder automatische Platzierung von Landmarken erleichtert. Eine automatische Platzierung von Landmarken kann bevorzugt mittels der zuvor beschriebenen Methoden zur Definition des Objektkoordinatensystems erfolgen.

Vorteil der Variante c) ist, dass die "Knoten" einmalig vorab mit den das Messkoordinatensystem definierenden Punkten oder Zwangsbedingungen verbunden werden müssen, und fortan für jede Messung automatisch das Messkoordinatensystem an der entsprechenden Geometrie des oder der 3D-Objekte 300` ausgerichtet ist.

Soll eine Ausgabe der Messwerte in einer Längeneinheit erfolgen, z.B. für Abstandsmessungen, muss die Röntgenaufnahme 100 bzw. das korrigierte DRR 524 auf einen Pixel/Längeneinheit-Maßstab kalibriert werden. Dies kann anhand eines auf der Röntgenaufnahme 100 sichtbaren Kalibrierobjekts mit bekannten Abmessungen, oder durch Übertragung des in der Regel bekannten Maßstabs des 3D-Modells (Voxel/Längeneinheit) erfolgen. Besteht das 3D-Modell aus einem ASM oder SSM, ist die Größe des Modells stets einer der Vektoren mit hoher Variabilität. In diesem Fall muss die Kalibrierung der Röntgenaufnahme, dem korrigierten DRR 524 in Ausgabeebene oder des ASM/AAM/SSM auf Basis eines auf der Röntgenaufnahme 100 sichtbaren Kalibrierobjektes mit bekannten Abmessungen erfolgen. Bei geringeren Anforderungen an die Genauigkeit kann auf statistische morphologische Daten sichtbarer Objekte oder den ungefähren Maßstab der Röntgenaufnahme 100 ausgewichen werden, der üblicherweise in der Datei der Röntgenaufnahme enthalten ist.

Winkelmessungen bedürfen üblicherweise keiner Kalibrierung auf einen Längenmaßstab, so dass dieser Schritt bei Messaufgaben, die ausschließlich Winkelmessungen erfordern, entfallen kann.

Die eigentliche Vermessung kann dann mit Hilfe des Vermessungsmoduls in einem weiteren Schritt unter Verwendung der korrigierten Projektionsrichtung 536 an dem korrigierten DRR 524 in der Ausgabeebene 600 erfolgen. Dies ist in Figur 6 illustriert. Aus der Figur ist ersichtlich, dass die Umrandung des Objekts im korrigierten DRR 524 keine Doppelkanten aufweist, so dass eine exakte Vermessung möglich ist. Insbesondere kann eine exakte Winkelmessung 140 und eine exakte Abstandsmessung 150 durchgeführt werden.

Alternativ kann die Kenntnis der Verkippung zusammen mit einer oder mehrerer Korrekturfunktionen genutzt werden, um die wahren Längen und Winkel zwischen den Messpunktdefinitionen, die in der unkorrigierten Röntgenaufnahme 100 erstellt wurden, rechnerisch zu korrigieren. Dies kann in diesem Fall ebenfalls durch das erfindungsgemäße Vermessungsmodul durchgeführt werden.

Als dritte vorteilhafte Variante bietet es sich an, die Messungen direkt im 3D-Modell durchzuführen. Hierfür werden Abstände und Winkel zwischen vordefinierten Landmarken der (ggf. transformierten und angepassten) 3D-Objekte 300 genutzt und die euklidischen Abstände bzw. die Abstandskomponenten bezogen auf das Messkoordinatensystem oder die Ausgabeebene 600 angegeben. Diese Variante bietet sich insbesondere bei mathematisch beschriebenen 3D-Objekten 300, basierend auf den Netzknoten an (z.B. bei ASM/AAM/SSM).

Ist eine dauerhafte Ergebnisspeicherung gewünscht, werden die Messwerte z.B. in eine Datenbank, ein PACS oder KIS-System, oder über einen ausdruckbaren Bericht gespeichert.

## Patentansprüche

1. Verfahren zur Vermessung einer Röntgenaufnahme (100) eines medizinischen Untersuchungsbereiches (400), der mindestens zwei Objekte (300) aufweist, mit den folgenden Schritten:
- Bereitstellen eines 3D-Modells (400') eines Untersuchungsbereichs (400), das zumindest zwei den zu vermessenden Objekten (300) zuzuordnende virtuelle 3D-Objekte (300`) umfasst,
- Berechnen eines digital rekonstruierten Röntgenbildes (520) anhand des 3D-Modells (400') und unter Annahme einer virtuellen Projektionsrichtung (540),
- Vergleichen der Röntgenaufnahme (100) mit dem digital rekonstruierten Röntgenbild (520),
- Verändern der virtuellen Projektionsrichtung (540) relativ zu den virtuellen 3D-Objekten (300') sowie der Position und/oder Ausrichtung der virtuellen 3D-Objekte (300') relativ zueinander,
- Wiederholen der Schritte des Vergleichens und des Veränderns bis eine virtuelle Projektionsrichtung (540) und für jedes der 3D-Objekte (300) eine Position und/oder eine relative Ausrichtung gefunden sind, bei denen eine maximale Übereinstimmung zwischen der Röntgenaufnahme (100) und dem digital rekonstruierten Röntgenbild (520) besteht,
- Ermitteln einer einem der virtuellen 3D-Objekte (300') zuzuordnenden Objektebene (192),
- Definieren einer korrigierten Projektionsrichtung (536) sowie einer zugehörigen Ausgabeebene (600), die eine bekannte Ausrichtung zu der ermittelten Objektebene (192) aufweist, wobei die korrigierte Projektionsrichtung (536) orthogonal zu der Ausgabeebene (600) ausgerichtet ist,
- Vermessen der Röntgenaufnahme (100) unter Berücksichtigung der Abweichung zwischen korrigierter virtueller Projektionsrichtung (536) und der gefundenen virtuellen Projektionsrichtung (540) und unter Berücksichtigung der ermittelten Positionen der virtuellen 3D-Objekte (300') innerhalb des 3D-Modells (400') und/oder der Ausrichtungen der virtuellen 3D-Objekte (300') relativ zu der korrigierten Projektionsrichtung (536).

2. Verfahren nach Anspruch 1, bei dem die Ausgabeebene (600) parallel zu der ermittelten Objektebene (192) ausgerichtet ist, oder bei dem die Ausgabeebene (600) parallel zu einer Hauptebene eines durch das 3D-Modell (400') oder durch eines der virtuellen 3D-Objekte (300') vorgegebenen Koordinatensystems ausgerichtet ist.

3. Verfahren nach Anspruch 1 oder 2, bei dem der Schritt des Vermessens der Röntgenaufnahme (100) folgendes umfasst:
- Ermitteln einer Korrekturfunktion, die sich aus der Abweichung zwischen korrigierter virtueller Projektionsrichtung (536) und gefundener virtueller Projektionsrichtung (540) ergibt,
- Anwenden der Korrekturfunktion auf die gewonnenen Messergebnisse.

4. Verfahren nach Anspruch 1 oder 2, bei dem anhand der korrigierten virtuellen Projektionsrichtung (536) ein korrigiertes digital rekonstruiertes Röntgenbild (524) in der Ausgabeebene (600) erzeugt wird, wobei die Röntgenaufnahme (100) unter Verwendung des korrigierten digital rekonstruierten Röntgenbildes (524) in der Ausgabeebene (600) vermessen wird.

5. Verfahren nach einem der Ansprüche 1 bis 4, bei dem der Schritt des Vermessens eine Messung eines Abstandes zwischen Messpunkten, eine Messung eines Winkels zwischen Messgeraden und/oder eine Messung eines Flächeninhalts von Messflächen umfasst, und/oder
bei dem ein Längenmaßstab des 3D-Modells (400') auf die Röntgenaufnahme (100) übertragen wird.

6. Verfahren nach einem der Ansprüche 1 bis 5, bei dem zumindest eines der virtuellen 3D-Objekte (300') durch ein Verfahren zur dreidimensionalen Bildgebung, insbesondere durch Computertomographie oder Magnetresonanztomographie, gewonnen wird.

7. Verfahren nach Anspruch 6, bei dem die virtuelle Objektebene (192) auf Basis eines der virtuellen 3D-Objekte (300') unter Verwendung von dem virtuellen 3D-Objekt (300') zugeordneten Grauwertintensitäten ermittelt wird, wobei die virtuelle Objektebene (192) bevorzugt auf Basis einer Optimierung, insbesondere einer Least-Square-Optimierung, des Abstandes zwischen Punkten, Geraden, Kurven oder Ebenen und einer Auswahl von Voxeln des 3D-Objekts (300') ermittelt wird und/oder auf Basis von Kanten oder Flächen des 3D-Objekts (300`) und/oder mit Methoden des maschinellen Lernens, insbesondere mit trainierten neuronalen Netzwerken ermittelt wird.

8. Verfahren nach einem der Ansprüche 1 bis 5, bei dem zumindest eines der virtuellen 3D-Objekte (300') auf einer computergenerierten Geometriebeschreibung basiert, insbesondere auf einem "Statistical Shape Modell", einem "Active Appearance Modell" oder einem "Active Shape Modell".

9. Verfahren nach Anspruch 8, bei dem die virtuelle Objektebene (192) ermittelt wird auf Basis von vorab mit einem der virtuellen 3D-Objekte (300') assoziierten Landmarken.

10. Verfahren nach einem der Ansprüche 8 oder 9, bei dem zumindest eines der virtuellen 3D-Objekte (300') zusätzlich durch lokale und/oder elastische Deformation verändert wird.

11. Verfahren nach einem der Ansprüche 1 bis 10, bei dem der Schritt des Vermessens die Messung eines Abstandes zwischen zwei Messpunkten und/oder die Messung eines Winkels zwischen zwei Messgeraden umfasst, wobei die beiden Messpunkte und/oder Messgeraden verschiedenen virtuellen 3D-Objekten (300') zuzuordnen sind.

12. Recheneinrichtung umfassend Mittel zur Durchführung eines Verfahrens nach einem der Ansprüche 1 bis 11, mit
- einem Rechenmodul, das zur Berechnung des digital rekonstruierten Röntgenbildes (520) unter Verwendung des 3D-Modells (400') und unter Annahme einer virtuellen Projektionsrichtung (540) ausgelegt ist, wobei das 3D-Modell (400')den Objekten (300) zuzuordnende virtuelle 3D-Objekte (300') umfasst,
- einem Vergleichsmodul zum Vergleichen der Röntgenaufnahme (100) mit dem digital rekonstruierten Röntgenbild (520),
- einem Veränderungsmodul, das zur Veränderung der virtuellen Projektionsrichtung (540) relativ zu den virtuellen 3D-Objekten (300') und zur Veränderung der Positionen und/oder der Ausrichtung der virtuellen 3D-Objekte (300') relativ zueinander ausgebildet ist,
- einem Geometriemodul, das zur Ermittlung der einem der virtuellen 3D-Objekte (300') zuzuordnenden virtuellen Objektebene (192) ausgebildet ist sowie zur Definition der Ausgabeebene (600), die eine bekannte Ausrichtung zu der Objektebene (192) aufweist und orthogonal zu der korrigierten Projektionsrichtung (536) ausgerichtet ist, und
- einem Vermessungsmodul zum Vermessen der Röntgenaufnahme (100) unter Berücksichtigung der Abweichung zwischen korrigierter virtueller Projektionsrichtung (536) und der gefundenen virtuellen Projektionsrichtung (540) und unter Berücksichtigung der ermittelten Positionen der virtuellen 3D-Objekte (300') innerhalb des 3D-Modells (400') und/oder der Ausrichtungen der virtuellen 3D-Objekte (300') relativ zu der korrigierten Projektionsrichtung (536).

13. Vorrichtung zur Vermessung einer Röntgenaufnahme (100) eines medizinischen Untersuchungsbereiches (400), mit einer Recheneinrichtung nach Anspruch 12 und mit einem Speicher, in dem das 3D-Modell (400') des Untersuchungsbereichs (400) abgelegt ist.

14. Computerprogramm zur Vermessung einer Röntgenaufnahme (100) eines medizinischen Untersuchungsbereiches (400), das einen Computerprogrammcode für die Durchführung eines Verfahrens nach einem der Ansprüche 1 bis 11 enthält.

15. System zur Vermessung einer Röntgenaufnahme, mit:
- einer Röntgenquelle (500), die dazu geeignet ist, Röntgenstrahlung entlang einer Projektionsrichtung (530) auf einen medizinischen Untersuchungsbereich (400) mit mindestens zwei Objekten (300) auszustrahlen,
- einem Röntgendetektor zum Detektieren einer in einer Bildebene erzeugten Röntgenaufnahme (100),
wobei das System eine Recheneinrichtung nach Anspruch 12 aufweist, in der eine Vermessung der erzeugten Röntgenaufnahme (100) unter Bereitstellung eines 3D-Modells (400') des Untersuchungsbereichs (400) aus einem Speicher erfolgt, wobei das 3D-Modell (400`)den zu vermessenden Objekten (300) zuzuordnende virtuelle 3D-Objekte (300') umfasst.

## Claims

1. A method for measuring an X-ray image (100) of a medical examination area (400) which has at least two objects (300), the method comprising:
providing a 3D model (400') of an examination area (400) which includes a at least two virtual 3D objects (300') to be assigned to the objects (300) to be measured;
computing a digitally reconstructed X-ray picture (520) based on the 3D model (400') and under an assumption of a virtual projection direction (540);
comparing the X-ray image (100) with the digitally reconstructed X-ray picture (520);
changing the virtual projection direction (540) relative to the virtual 3D objects (300') as well as the position and/or orientation of the virtual 3D objects (300') relative to each other;
repeating the steps of comparing and changing until a virtual projection direction (540) and a position and/or relative orientation for each of the 3D objects (300) are found for which a maximum correlation between the X-ray image and the digitally reconstructed X-ray picture is given;
determining an object plane (192) to be assigned to one of the virtual 3D objects (300');
defining a corrected projection direction (536) and an associated output plane (600) which has a known orientation with respect to the object plane (192) determined, the corrected projection direction (536) being oriented orthogonally to the output plane (600); and
measuring the X-ray image (100), taking into account a deviation between the corrected virtual projection direction (536) and the found virtual projection direction (540) and taking into account the positions determined of the virtual 3D objects (300') within the 3D model (400') and/or the orientations of the virtual 3D objects (300') relative to the corrected projection direction (536).

2. The method according to claim 1, wherein output plane (600) is oriented parallel to the object plane (192), or wherein the output plane (600) is oriented parallel to a principal plane of a coordinate system predefined by the 3D model (400') or by one of the virtual 3D objects (300').

3. The method according to claim 1 or 2, wherein the step of measuring the X-ray image (100) includes
- determining a correction function that results from the deviation between the corrected virtual projection direction (536) and the found virtual projection direction (540),
- applying the correction function to the measurement results.

4. The method according to claim 1 or 2, wherein a corrected digitally reconstructed X-ray picture (524) in the output plane (600) is generated on the basis of the corrected virtual projection direction (536), wherein the X-ray image (100) is measured using the corrected digitally reconstructed X-ray picture (524) in the output plane (600).

5. The method according to claim any one of claims 1 to 4, wherein the step of measuring includes a measurement of a distance between measuring points, a measurement of an angle between measuring lines, and/or a measurement of a surface area of measuring areas; and/or
wherein a scale of length of the 3D model (400') is transferred to the X-ray image (100).

6. The method according to any one of claims 1 to 5, wherein at least one of the virtual 3D objects (300') is obtained by a method for three-dimensional imaging or by computed tomography or magnetic resonance imaging.

7. The method according to claim 6, wherein the virtual object plane (192) is determined on the basis of one of the virtual 3D objects (300') using gray-scale intensities assigned to the virtual 3D object (300'), wherein the virtual object plane (192) preferably is determined on the basis of an optimization, in particular a least squares optimization, of the distance between points, lines, curves, or planes, and a selection of voxels of the 3D object (300'), and/or is determined on the basis of edges or surfaces of the 3D object (300') and/or with machine learning methods, especially with trained neural networks.

8. The method according to any one of claims 1 to 5, wherein at least one of the virtual 3D objects (300') is based on a computer-generated geometry description or on a statistical shape model, an active appearance model, or an active shape model.

9. The method according to claim 8, wherein the virtual object plane (192) is determined based on landmarks associated in advance with one of the virtual 3D objects (300').

10. The method according to claim 8 or 9, wherein at least one of the virtual 3D objects (300') is additionally modified through local and/or elastic deformation.

11. The method according to any one of claims 1 to 10, wherein the measuring step includes the measurement of a distance between two measuring points and/or the measurement of an angle between two measuring lines, and wherein the two measuring points and/or measuring lines are to be assigned to different virtual 3D objects (300').

12. A computing device comprising means for carrying out a method according to any one of claims 1 to 11, the device comprising:
a computing module which is designed to compute the digitally reconstructed X-ray picture (520) using the 3D model (400') and under the assumption of a virtual projection direction (540), wherein the 3D model (400') comprises virtual 3D objects (300') to be assigned to the objects (300);
a comparison module for comparing the X-ray image (100) with the digitally reconstructed X-ray picture (520);
a change module, which is designed to change the virtual projection direction (540) relative to the virtual 3D objects (300') and to change the positions and/or the orientations of the virtual 3D objects (300') relative to one another;
a geometry module, which is designed to determine the virtual object plane (192) to be assigned to one of the virtual 3D objects (300') and to define the output plane (600) which has a known orientation to the object plane (192) and is oriented orthogonally to the corrected projection direction (536); and
a measurement module for measuring the X-ray image (100), taking into account the deviation between the corrected virtual projection direction (536) and the found virtual projection direction (540) and taking into account the positions of the virtual 3D objects (300') within the 3D model (400') determined in step e. and/or the orientations of the virtual 3D objects (300') relative to the corrected projection direction (536).

13. A device for measuring an X-ray image (100) of a medical examination area (400), comprising a computing device according to claim 12, and comprising a memory, in which the 3D model (400') of the examination area is stored.

14. A computer program for measuring an X-ray image (100) of a medical examination area (400), the computer program comprising computer program code for carrying out a method according to any one of claims 1 to 11.

15. An X-ray measuring system comprising:
an X-ray source (500) adapted to emit X-radiation along a projection direction (530) onto an examination area (400) with at least two objects (300);
an X-ray detector detecting an X-ray image (100) produced in an image plane;
wherein the system comprises a computing device according to claim 12, in which measuring of the produced X-ray image (100) is made by providing a 3D model (400') of the examination area (400) from a memory, the 3D model including virtual 3D objects (300') to be assigned to the objects (300) to be measured.

## Revendications

1. Procédé de mesure d'une radiographie (100) d'une zone d'examen médical (400) présentant au moins deux objets (300), comprenant les étapes suivantes consistant à :
- fournir un modèle 3D (400') d'une zone d'examen (400), qui comprend au moins deux objets 3D virtuels (300') à associer aux objets (300) à mesurer,
- calculer une image radiographique reconstruite numériquement (520) en utilisant le modèle 3D (400') et en supposant une direction de projection virtuelle (540),
- comparer la radiographie (100) avec l'image radiographique reconstruite numériquement (520),
- modifier la direction de projection virtuelle (540) par rapport aux objets 3D virtuels (300'), et modifier la position et/ou l'orientation des objets 3D virtuels (300') l'un par rapport à l'autre,
- répéter les étapes de comparaison et de modification jusqu'à ce qu'une direction de projection virtuelle (540) et, pour chacun des objets 3D (300), une position et/ou une orientation relative soient trouvées, auxquelles il existe une concordance maximale entre la radiographie (100) et l'image radiographique reconstruite numériquement (520),
- déterminer un plan d'objet (192) à associer à l'un des objets 3D virtuels (300'),
- définir une direction de projection corrigée (536) ainsi qu'un plan de sortie associé (600) qui présente une orientation connue par rapport au plan d'objet déterminé (192), la direction de projection corrigée (536) étant orientée orthogonalement par rapport au plan de sortie (600),
- mesurer la radiographie (100) en tenant compte de l'écart entre la direction de projection virtuelle corrigée (536) et la direction de projection virtuelle trouvée (540), et en tenant compte des positions déterminées des objets 3D virtuels (300') au sein du modèle 3D (400') et/ou des orientations des objets 3D virtuels (300') par rapport à la direction de projection corrigée (536).

2. Procédé selon la revendication 1,
dans lequel le plan de sortie (600) est orienté parallèlement au plan d'objet déterminé (192), ou
le plan de sortie (600) est orienté parallèlement à un plan principal d'un système de coordonnées prédéfini par le modèle 3D (400') ou par l'un des objets 3D virtuels (300').

3. Procédé selon la revendication 1 ou 2,
dans lequel l'étape de mesure de la radiographie (100) consiste à :
- déterminer une fonction de correction résultant de l'écart entre la direction de projection virtuelle corrigée (536) et la direction de projection virtuelle trouvée (540),
- appliquer la fonction de correction aux résultats de mesure obtenus.

4. Procédé selon la revendication 1 ou 2,
dans lequel, à l'aide de la direction de projection virtuelle corrigée (536), une image radiographique reconstruite numériquement (524) corrigée est générée dans le plan de sortie (600), la radiographie (100) étant mesurée en utilisant l'image radiographique reconstruite numériquement (524) corrigée dans le plan de sortie (600).

5. Procédé selon l'une des revendications 1 à 4,
dans lequel l'étape de mesure comprend une mesure d'une distance entre des points de mesure, une mesure d'un angle entre des lignes droites de mesure et/ou une mesure d'une superficie de surfaces de mesure, et/ou
une échelle de longueur du modèle 3D (400') est transférée à la radiographie (100).

6. Procédé selon l'une des revendications 1 à 5,
dans lequel l'un au moins des objets 3D virtuels (300') est obtenu par un procédé d'imagerie tridimensionnelle, en particulier par tomographie assistée par ordinateur ou par imagerie par résonance magnétique.

7. Procédé selon la revendication 6,
dans lequel le plan d'objet virtuel (192) est déterminé sur la base de l'un des objets 3D virtuels (300') en utilisant des intensités de niveaux de gris associées à l'objet 3D virtuel (300'), le plan d'objet virtuel (192) étant déterminé de préférence en se basant sur une optimisation, en particulier une optimisation des moindres carrés, de la distance entre des points, des lignes droites, des courbes ou des plans, et sur une sélection de voxels de l'objet 3D (300'), et/ou en se basant sur des arêtes ou des surfaces de l'objet 3D (300') et/ou avec des méthodes d'apprentissage automatique, en particulier avec des réseaux neuronaux entraînés.

8. Procédé selon l'une des revendications 1 à 5,
dans lequel l'un au moins des objets 3D virtuels (300') est basé sur une description géométrique générée par ordinateur, en particulier sur un "Statistical Shape Modell (modèle statistique de forme)", un "Active Appearance Modell (modèle d'apparence active)" ou un "Active Shape Modell (modèle de forme active)".

9. Procédé selon la revendication 8,
dans lequel le plan d'objet virtuel (192) est déterminé en se basant sur des repères préalablement associés à l'un des objets 3D virtuels (300').

10. Procédé selon l'une des revendications 8 ou 9,
dans lequel l'un au moins des objets 3D virtuels (300') est en supplément modifié par déformation locale et/ou élastique.

11. Procédé selon l'une des revendications 1 à 10,
dans lequel l'étape de mesure comprend la mesure d'une distance entre des points de mesure et/ou la mesure d'un angle entre deux lignes droites de mesure, les deux points de mesure et/ou lignes droites de mesure étant à associer à différents objets 3D virtuels (300').

12. Dispositif de calcul comprenant des moyens pour mettre en oeuvre un procédé selon l'une des revendications 1 à 11, comprenant
- un module de calcul conçu pour calculer l'image radiographique reconstruite numériquement (520) en utilisant le modèle 3D (400') et en supposant une direction de projection virtuelle (540), le modèle 3D (400') comprenant des objets 3D virtuels (300') à associer aux objets (300),
- un module de comparaison pour comparer la radiographie (100) avec l'image radiographique reconstruite numériquement (520),
- un module de modification conçu pour modifier la direction de projection virtuelle (540) par rapport aux objets 3D virtuels (300') et pour modifier les positions et/ou les orientations des objets 3D virtuels (300') les uns par rapport aux autres,
- un module de géométrie conçu pour déterminer le plan d'objet virtuel (192) à associer à l'un des objets 3D virtuels (300') et pour définir le plan de sortie (600) qui présente une orientation connue par rapport au plan d'objet (192) et qui est orienté orthogonalement à la direction de projection corrigée (536), et
- un module de mesure pour mesurer la radiographie (100) en tenant compte de l'écart entre la direction de projection virtuelle corrigée (536) et la direction de projection virtuelle trouvée (540) et en tenant compte des positions déterminées des objets 3D virtuels (300') au sein du modèle 3D (400') et/ou des orientations des objets 3D virtuels (300') par rapport à la direction de projection corrigée (536).

13. Dispositif de mesure d'une radiographie (100) d'une zone d'examen médical (400), comprenant un dispositif de calcul selon la revendication 12 et une mémoire dans laquelle est stocké le modèle 3D (400') de la zone d'examen (400).

14. Programme informatique de mesure d'une radiographie (100) d'une zone d'examen médical (400), comprenant un code de programme informatique pour mettre en oeuvre un procédé selon l'une des revendications 1 à 11.

15. Système de mesure d'une radiographie, comprenant :
- une source de rayons X (500) adaptée pour émettre des rayons X le long d'une direction de projection (530) sur une zone d'examen médical (400) présentant au moins deux objets (300),
- un détecteur de rayons X pour détecter une radiographie (100) générée dans un plan d'image,
le système étant relié à un dispositif de calcul selon la revendication 12, dans lequel une mesure de la radiographie (100) générée est effectuée en fournissant un modèle 3D (400') de la zone d'examen (400) à partir d'une mémoire, le modèle 3D (400') comprenant des objets 3D virtuels (300') à associer aux objets (300) à mesurer.
